(19) **Europäisches Patentamt / European Patent Office / Office européen des brevets**

(11) **EP 2 139 586 B1**

(12) **FASCICULE DE BREVET EUROPEEN**

(45) Date de publication et mention
de la délivrance du brevet:
**06.11.2013 Bulletin 2013/45**

(21) Numéro de dépôt: **08787985.4**

(22) Date de dépôt: **13.03.2008**

(51) Int Cl.:
**B01D 53/04** $^{(2006.01)}$     **A61M 16/00** $^{(2006.01)}$

(86) Numéro de dépôt international:
**PCT/FR2008/050424**

(87) Numéro de publication internationale:
**WO 2008/135675 (13.11.2008 Gazette 2008/46)**

(54) **PROCEDE POUR ELIMINER LE N2O D'UN FLUX GAZEUX**

VERFAHREN ZUR ENTFERNUNG VON N2O AUS EINEM GASSTROM

METHOD FOR REMOVING N2O FROM A GASEOUS FLOW

(84) Etats contractants désignés:
**AT BE BG CH CY CZ DE DK EE ES FI FR GB GR
HR HU IE IS IT LI LT LU LV MC MT NL NO PL PT
RO SE SI SK TR**

(30) Priorité: **16.03.2007 FR 0753889**

(43) Date de publication de la demande:
**06.01.2010 Bulletin 2010/01**

(73) Titulaires:
• **L'Air Liquide Société Anonyme pour l'Etude et
l'Exploitation des Procédés Georges Claude
75007 Paris (FR)**
• **Air Liquide Santé (International)
75007 Paris (FR)**

(72) Inventeur: **MOREAU, Serge**
**F-78140 Velizy Villacoublay (FR)**

(74) Mandataire: **Pittis, Olivier
L'Air Liquide, S.A.,
Direction de la Propriété Intellectuelle,
75, Quai d'Orsay
75321 Paris Cedex 07 (FR)**

(56) Documents cités:
**EP-A- 1 005 904      EP-A1- 0 995 477
WO-A-02/26355      US-A- 4 355 637**

## Description

L'invention porte sur un procédé de purification de mélanges gazeux contenant principalement du protoxyde d'azote, de l'azote et de l'oxygène et éventuellement des impuretés, en particulier l'eau et le dioxyde de carbone, notamment un flux gazeux expiré par un patient recevant un mélange gazeux de $N_2O$ et d'$O_2$.

Le protoxyde d'azote ($N_2O$), aussi appelé gaz hilarant ou oxyde nitreux a de nombreuses utilisations.

Il est notamment utilisé en anesthésie et en analgésie pour le traitement de la douleur, par exemple en mélange à environ 50% en volume de $N_2O$ et 50% en volume de $O_2$.

En anesthésie et en analgésie, le mélange gazeux riche en $N_2O$ est d'abord inhalé par le patient, puis une partie importante du $N_2O$ se retrouve dans les gaz expirés par le patient, en mélange notamment avec de fortes proportions de $CO_2$ et de vapeur d'eau.

Ainsi, pour un mélange volumique à 50% de $O_2$ et 50% de $N_2O$ qui est inhalé par un patient, les gaz expirés par ce patient contiennent du $N_2O$ saturé en humidité, environ 4% en volume de $CO_2$ et plus de 40 % en volume d'oxygène.

Or, il convient d'éliminer le $N_2O$ des gaz expirés par le patient car il risque de se retrouver dans l'air intérieur des bâtiments et de s'y accumuler au fil du temps.

En effet, empêcher une telle accumulation de $N_2O$ dans les bâtiments hospitaliers est primordial car des effets indésirables ont été notés lors d'inhalation intense et fréquente de $N_2O$, tel qu'une carence en vitamine B12 chez les personnes exposées.

Une récupération et une élimination de $N_2O$ par catalyse a déjà été proposée, notamment par les documents US-A-4259303, WO-A-9925461, US-A-2006008401, FR-A-2773144, JP-A-2006142160, EP-A-0698411 et JP-A-10165818.

Cependant, cette solution est compliquée car elle implique l'utilisation de composés de métaux de transition dont certains sont coûteux et pas faciles à préparer. Par ailleurs, les impuretés présentes dans le gaz à traiter peuvent empoisonner et dénaturer le catalyseur. De plus, ces procédés imposent un chauffage à plusieurs centaines de °C avec toutes les complications et la consommation d'énergie associées.

En outre, le document EP-A-0 995 477 propose un procédé pour adsorber uniquement de faibles proportions de $N_2O$ présent dans l'air atmosphérique, à savoir de l'ordre de quelques ppm en volume. Toutefois, ce document ne préconise aucune solution permettant d'éliminer le N2O lorsqu'il est présent en une proportion volumique de plusieurs %, voire de plusieurs dizaine de %.

Le document US 4,355,637 enseigne l'utilisation d'un masque dans les salles d'opération des hôpitaux contenant l'air expiré par des patients sous anesthésie ; ledit masque comprenant une couche de zéolite pour éliminer le $N_2O$ expiré par le patient. Toutefois, cette solution ne semble pas assez efficace pour éliminer le $N_2O$ expiré par le patient.

Par ailleurs, le document WO-A-02/26355 enseigne un procédé et un dispositif servant à traiter les gaz anesthésiques rejetés par les patients qui contiennent des teneurs en $N_2O$ comprise entre 3 et 70 % en volume. Dans ce cas, les gaz sont mis en contact avec un catalyseur servant à détruire les molécules de $N_2O$ à une température entre 200 et 600°C.

De là, il n'a jusqu'à présent pas été possible d'éliminer de façon suffisamment efficace le protoxyde d'azote expiré par le patient et qui se retrouve dans les salles de soins ou analogues des bâtiments hospitaliers.

Au vu de cela, un problème qui se pose est d'améliorer les procédés de traitement de flux gazeux d'alimentation contenant du protoxyde d'azote en une proportion volumique d'au moins 5%, préférentiellement d'au moins 10 à 20% en volume, en particulier de mélanges gazeux contenant du $N_2O$, de l'oxygène, de la vapeur d'eau et quelques % de $CO_2$, de manière à pouvoir accroître l'élimination du $N_2O$ expiré par les patients au sein des bâtiments hospitaliers ou analogues.

Une solution selon l'invention est un procédé de purification d'un flux gazeux d'alimentation expiré par un être humain ou un animal contenant au moins 20% en volume de $N_2O$ et de l'oxygène, dans lequel :

(a) on met en contact le flux gazeux, à une température comprise entre 20°C et 40°C et à une pression comprise entre 0,80 bars et 1,30 bars, avec un adsorbant principal comprenant au moins une zéolite échangée à plus de 50% par un ou des cations métalliques, présentant une cinétique d'adsorption comprise entre 2 et 20 sec$^{-1}$ déterminée à 25°C et 1 bar par mesure de la courbe de percée à partir d'un mélange $O_2/N_2O$ contenant moins de 10% en volume de $N_2O$, avec constante cinétique selon la Linear Driving Force, et une capacité d'adsorption du $N_2O$, mesurée à 1 bar et à 20°C, supérieure à 80 Ncm$^3$/g de manière à adsorber au moins une partie du $N_2O$ dudit flux et à produire un flux gazeux purifié, et

(b) on récupère un flux gazeux purifié contenant une teneur en $N_2O$ inférieure à la teneur en $N_2O$ du flux gazeux d'alimentation mis en contact avec l'adsorbant principal à l'étape a).

Selon le cas, le procédé selon l'invention peut comprendre l'une des caractéristiques suivantes :

- le flux gazeux d'alimentation, mis en contact avec l'adsorbant principal à l'étape a), contient au moins 30 % en volume de $N_2O$, de préférence au moins 40% en volume de $N_2O$ ;
- qu'à l'étape (b) on récupère un flux gazeux purifié contenant moins de 10% en volume de $N_2O$, de préférence moins de 5% en volume de $N_2O$, plus préférentiellement moins de 3% en volume de $N_2O$ ;
- le flux gazeux d'alimentation mis en contact avec l'adsorbant principal à l'étape a) contient au moins 30% en volume, de préférence au moins 40% en volume, de N$_2$O et à l'étape b), on récupère un flux gazeux purifié contenant moins de 10 % en volume de N$_2$O, de préférence moins de 5% en volume de N$_2$O ;

- la teneur le flux gazeux d'alimentation mis en contact avec l'adsorbant principal à l'étape a) contient moins de 80% en volume de N$_2$O, de préférence moins de 70% en volume de N$_2$O.

- l'adsorbant principal est constitué de particules de taille moyenne comprise entre 0,5 et 5 mm ; cette dimension correspondant soit à un diamètre lorsque les particules sont sphériques, soit à la plus grande longueur lorsque les particules sont notamment ellipsoïdales ;

- l'adsorbant principal a une cinétique d'adsorption comprise entre 2 et 10 sec$^{-1}$.

La cinétique est mesurée à 25°C et 1 bar de pression totale. On mesure la courbe de percée à partir d'un mélange 02 contenant moins de 10% de N2O. La constante cinétique est celle de la LDF (Linear driving force).

- le flux gazeux d'alimentation en traversant l'adsorbant principal de l'étape a) subit une perte de charge inférieure à 20 mbars, de préférence inférieure à 5 mbars ;

- la zéolithe est de type A, X, LSX, Mordénite, Offretite, Chabazite, Clinoptilolite ou Erionite ;

- la zéolithe est échangée par un ou plusieurs cations métalliques choisis parmi Na$^+$, Li$^+$, K$^+$, Ca$^{2+}$, Mg$^{2+}$ et Ba$^{2+}$, et associés ou non à des métaux de transition, de préférence choisis parmi l'argent, le zinc ou le cuivre ;

- l'adsorbant principal a une capacité d'adsorption à 1 bar et à 20°C du N$_2$O supérieure à 90 Ncm$^3$/g ;

- le flux gazeux d'alimentation contient également de la vapeur d'eau, du CO$_2$ et/ou de l'argon et/ou de l'azote et est de préférence expiré par un patient ;

- la capacité d'adsorption du CO$_2$ de l'adsorbant principal mesuré à 0.04 bar et à 20°C est supérieure ou égale à 30 Ncm$^3$/g, de préférence supérieure à 50 Ncm$^3$/g ;

- le procédé comporte, en outre, préalablement à l'étape a), une mise en contact du flux gazeux d'alimentation avec un adsorbant secondaire comprenant un dessicant apte à piéger tout ou partie de la vapeur d'eau contenue dans le flux gazeux, de préférence de l'alumine ;

- le procédé comporte, en outre, l'étape de régénérer l'adsorbant sous vide, c'est à dire à une pression comprise entre 10 et 300 mbars, par balayage d'un gaz sec et inerte, chauffé à une température comprise entre 80 et 250°C, de préférence entre 125 et 200°C ;

- le débit du flux gazeux d'alimentation à purifier est compris entre 1 et 30 Nlitres/min, de préférence entre 8 et 15 Nlitres/min ;

Dans le cadre de l'invention, on décrit aussi une méthode d'anesthésie inhalatoire d'un être humain ou d'un animal, dans laquelle :

i) on administre par inhalation à un être humain ou à un animal, un mélange gazeux anesthésique contenant de l'oxygène et une proportion de N$_2$O supérieure à 30% en volume,
ii) on récupère au moins une partie des gaz expirés par ledit humain ou animal,
iii) on soumet tout ou partie des gaz expirés récupérés à l'étape ii), à un procédé de purification selon l'invention.

Selon le cas, la méthode d'anesthésie décrite ci-dessus peut comprendre l'une ou plusieurs des caractéristiques suivantes :

- l'être humain est un patient,
- le mélange gazeux anesthésique contient uniquement de l'oxygène et du N$_2$O,
- le mélange gazeux anesthésique est formé de 50% de N$_2$O et de 50% de O$_2$, en volume.
- le mélange gazeux anesthésique contient une proportion suffisante de N$_2$O pour induire ou maintenir une anesthésie chez ledit humain ou animal,
- le mélange gazeux anesthésique est administré aux voies aériennes dudit humain ou animal, par exemple au moyen d'un masque ou analogue préférentiellement relié à un ventilateur d'anesthésie ou à tout appareil d'administration de gaz apte à et conçu pour alimenter le patient en gaz anesthésique.

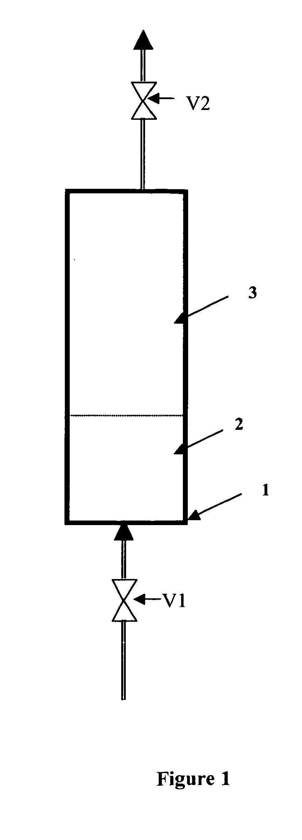

L'invention va être décrite plus en détail en références à la figure 1 donnée à titre illustratif.
Le gaz à purifier est un mélange gazeux ayant la composition des gaz expirés par un patient subissant une anesthésie inhalatoire au moyen d'un mélange gazeux formé de 50% N$_2$O et de 50% de 02 (% en volume) environ.
Le gaz à purifier contenant du N$_2$O, du CO$_2$, de la vapeur d'eau et de l'oxygène, voire de l'argon et/ou de l'azote, est récupéré puis dirigé via la vanne V1 vers l'adsorbeur 1 constitué d'un lit amont d'alumine 2, dans lequel est retenue l'eau, suivi d'un lit aval de zéolite, par exemple une zéolite 13 X, dans lequel sont retenus par adsorption le N$_2$O et le CO$_2$. Le gaz purifié constitué principalement d'oxygène est collecté, en sortie d'adsorbeur, via la vanne V2, et peut être soit recyclé, soit rejeté à l'atmosphère.
A titre d'exemple, un respirateur et un humidificateur couplés à un poumon artificiel ont été utilisés pour simuler un patient. Le gaz à purifier a la composition suivante : 4,25 % de CO$_2$, 46,25% de O$_2$, et 49,5% de N$_2$O.

---

Le gaz est envoyé vers le respirateur avec les réglages suivants : 10 1/min, 15 minutes par cycle. Le gaz sortant du respirateur est envoyé dans l'humidificateur, puis dans le poumon artificiel, réglé aux caractéristiques d'un patient adulte (volume 600 ml, résistance 5 mbars/(1/s), compliance 23 ml/mbar). La sortie du poumon artificiel est reliée à l'adsorbeur. L'adsorbeur est rempli de 15 kg de zéolithe du type ZEOCHEM Z10-02 ND, taille de billes 1.6-2.6 mm. La zéolithe a été activée pendant une nuit vers 325°C sous flux d'azote sec, avec un débit pour renouveler le volume environ 10 fois par heures. Elle contient 100 % de cations Na+. La température de la pièce est comprise entre 17°C et 20°C. Le gaz sortant de l'adsorbeur est analysé quantitativement par analyse infrarouge à transformer de Fourier (FT-IR).

Le fonctionnement a consisté à simuler des "actes" médicaux d'une durée de 15 minutes, avec 15 minutes d'arrêt entre chaque.

Deux essais on été réalisés, avec, respectivement un adsorbeur de forme cylindrique et un adsorbeur de section en « fer à cheval » correspondant au système commercial. Pas de différence significative n'a été observée entre les deux géométries. Pendant les 12 premiers actes, aucune trace de $N_2O$ n'a été détecté en sortie, et au 13ème acte la percé en N2O a été observée. Le débit a alors été maintenu tel quel, et il a fallu 25 minutes pour retrouver en sortie la composition en entrée.

Il apparaît que, rapporté à la quantité de zéolithe, les deux géométries d'adsorbeur donnent des résultats comparables, correspondant à une capacité d'adsorption pratique d'environ 60 Nl/kg. L'isotherme d'adsorption en corps pur de la zeolithe utilisée est de 110 Nl/kg à 20 °C et 1 bar. La différence entre capacité pratique et en corps pur provient de la coadsorption avec l'eau et le $CO_2$, ainsi que de la longueur du front d'adsorption qui oblige à arrêter dès les premières traces en sortie.

La capacité d'adsorption de la zéolithe doit être située entre 80 $Ncm^3/g$ et 120 $Ncm^3/g$ à 1 bar et 20°C en corps pur, car c'est pour ces valeurs que la quantité d'adsorbant est adaptée à la forme et au volume de la bouteille. En effet, la bouteille utilisée est capable de contenir 1500 N1 de mélange, soit dix actes : dans l'intervalle de capacité revendiquée, on ne doit pas utiliser plus de 20 kg d'adsorbant, à cause du poids, et il n'est pas avantageux d'utiliser moins de 12 kg d'adsorbant car le volume d'adsorbant doit rester comparable avec celui de la bouteille et de son équipement.

Les différents lits d'adsorbants "pollués" peuvent ensuite être stockés dans l'adsorbeur fermé à la fin de l'intervention, en attente de régénération. Les lits "pollués" sont ensuite régénérés à contre-courant, à l'aide d'un gaz de régénération chauffé à une température comprise entre 80 et 250°C, de préférence entre 125 °C et 200°C. Le protoxyde d'azote, le dioxyde de carbone et l'eau sont ainsi désorbés des différents lits d'adsorbants et récupérés.

Le protoxyde d'azote est alors ensuite éliminé par mise à l'air à l'extérieur du bâtiment, détruit ou réutilisé après purification et stérilisation.

Le protoxyde d'azote désorbé, pendant la phase de régénération, est sous forme concentrée puisqu'il représente de 50% à 90% en volume du flux gazeux issu de la désorption des adsorbants.

Un séjour d'environ 1 heure des adsorbants à la température de régénération est suffisant pour désorber lesdits adsorbants.

De préférence, la régénération est réalisée au sein même de l'adsorbeur mais on peut envisager une régénération à l'extérieur de l'adsorbeur par manipulation directe des adsorbants. Le gaz de régénération est de préférence sec, c'est-à-dire qu'il ne contient pas d'eau à une teneur de plus de 10 ppm, de préférence pas plus de 1 ppm. Il est non réactif vis-à-vis des adsorbants. Ce peut être de l'air, de l'oxygène, de l'azote ou bien un mélange oxygène-azote de composition variable.

De manière alternative, la régénération peut être effectuée en mettant l'adsorbant sous vide dynamique, à une pression inférieure à 0.1 mbars, de préférence inférieure à 0.01 mbars, les autres conditions restant inchangées.

Après régénération, l'adsorbant est laissé à refroidir à l'intérieur ou à l'extérieur de l'adsorbeur, puis stocké dans l'adsorbeur fermé.

Plus généralement, la pression du flux gazeux expiré par le patient, c'est à dire des gaz expirés, est généralement comprise entre 970 mbars et 1080 mbars absolus. Ces gaz expirés par le patient sont récupérés par exemple au moyen d'un masque ou analogue, puis traités directement par mis en contact de ou des adsorbants comme expliqué ci-avant, ou éventuellement après filtration et/ou compression à une pression inférieure à 1.5 bars absolus.

La température du flux de gaz d'alimentation entrant dans la colonne d'adsorption est dans la plage 5 à 45 °C, de préférence 15 à 40 °C. De là, la température de fonctionnement de l'adsorbeur 1 au cours des étapes (a) et (b) du procédé de l'invention est également comprise entre 5 et 45 °C, de préférence entre 15°C et 40 °C, et encore plus préférentiellement entre 25 °C et 35 °C.

Il est préférable de maximiser l'adsorption de $CO_2$, bien que celui-ci entre en compétition avec le $N_2O$. En effet, le $CO_2$ s'adsorbe plus fortement que le $N_2O$ et en empêche l'adsorption. Il importe donc de minimiser la zone d'adsorbant saturé en $CO_2$, ce qui revient à maximiser la quantité de $CO_2$ stockée rapportée à la masse d'adsorbant.

Un seul lit d'adsorbant contenant de la zéolite peut être utilisé dans le cadre de l'invention. Cependant, la vapeur d'eau pouvant être éliminée par la zéolite elle-même, gêne l'adsorption du $CO_2$ et à fortiori, celle du $N_2O$.

De là, un lit d'alumine, placé en amont du lit contenant de la zéolite, permet de sécher au moins partiellement le flux gazeux d'alimentation, c'est-à-dire d'éliminer au moins une partie de l'eau qu'il véhicule. Bien que l'alumine soit préférée, d'autres matériaux dessicants, c'est-à-dire aptes à piéger la vapeur d'eau, comme le gel de silice ou des zéolites peuvent être utilisés.

Le débit à traiter peut-être intermittent, consistant par exemple en au plus 60 % du temps. En effet, le flux gazeux expiré par les patients est à débit variable du fait de la respiration.

L'adsorbant principal, selon l'invention, peut comprendre des grains ou particules d'adsorbant dont la phase solide adsorbante proprement dite est « aérée » par une porosité dispersée en volume qui assure le transport des gaz.

Or, la porosité peut être définie suivant l'expression : $q = \varepsilon * C_p + (1-\varepsilon) * C_s$, où $\varepsilon$ représente la porosité du grain adsorbant, q la concentration totale dans les grains d'adsorbant en mole/m$^3$, Cp la concentration en mole/m$^3$ dans la phase gazeuse de la porosité et $C_s$ la concentration dans la phase solide adsorbante en mole/m$^3$.

D'autre part, on a $\dfrac{dq}{dt} = a_k \times \left( \overline{C_p} - C_p \right),$

où $a_k$ représente la cinétique d'adsorption et $\overline{C_p}$ la concentration gazeuse qui correspondrait à l'équilibre avec les grains d'adsorbant.

De là, une porosité dispersée en volume qui assure le transport des gaz correspond à une cinétique d'adsorption de l'adsorbant principal comprise entre 2 et 20 sec$^{-1}$, de préférence inférieure à 10 sec$^{-1}$. Ceci est contraire à l'état de la technique qui cherche une cinétique d'adsorption maximale. Habituellement, on cherche à avoir un front d'adsorption aussi court que possible, pour retarder la sortie de l'impureté adsorbée.

Par ailleurs, la forte proportion de N$_2$O (supérieure à 30%, voire 40 %) raccourcit spontanément le front d'adsorption par effet « front de choc » résultant de la courbure de l'isotherme. En effet, lorsque l'isotherme d'adsorption est convexe, le front d'adsorption va avoir tendance à être le plus vertical possible, car les parties à haute concentration progressent plus vite que celles à basse concentration et les rattrapent donc.

La cinétique d'adsorption est importante car elle détermine la longueur du front d'adsorption dans l'adsorbant et conséquemment le taux d'utilisation de l'adsorbant. On entend par taux d'utilisation, le rapport de la capacité d'adsorption réalisée sur la capacité d'adsorption théorique.

De là, le taux d'utilisation selon l'invention doit être égale de préférence à environ $^2/_3$ pour N$_2$O, ¼ pour CO$_2$, et ⅛ pour l'eau.

D'autre part, en échange d'une contrainte sur la cinétique moins forte, on obtient une perte de charge diminuée car il est possible d'utiliser des particules plus grandes et/ou un adsorbant plus dense.

Ainsi, la perte de charge doit toujours être inférieure à 20 mbars, de préférence inférieure à 5 mbars, et la taille des particules d'adsorbant, qui détermine à la fois la cinétique d'adsorption et la perte de charge, doit être comprise entre 0.5 mm et 5 mm de diamètre équivalent en vue d'assurer un bon compromis entre ces deux facteurs. Les particules peuvent être sous forme de billes, d'extrudés ou de concassé, de préférence de billes.

On notera que la configuration de l'adsorbeur peut être cylindrique, à flux radial ou axial, ou bien encore d'une géométrie compacte avec, éventuellement, des éléments internes (chicanes) destinés à faciliter le contact entre le gaz et l'adsorbant.

## Revendications

1. Procédé de purification d'un flux gazeux d'alimentation expiré par un être humain ou un animal contenant au moins 20 % en volume de N$_2$O et de l'oxygène, dans lequel :

   (a) on met en contact le flux gazeux d'alimentation, à une température comprise entre 20°C et 40°C et à une pression comprise entre 0,80 bars et 1,30 bars, avec un adsorbant principal, comprenant au moins une zéolite échangée à plus de 50% par un ou des cations métalliques, présentant une cinétique d'adsorption comprise entre 2 et 20 sec$^{-1}$ déterminée à 25°C et 1 bar par mesure de la courbe de percée à partir d'un mélange O$_2$/N$_2$O contenant moins de 10% en volume de N$_2$O, avec constante cinétique selon la Linear Driving Force, et une capacité d'adsorption du N$_2$O, mesurée à 1 bar et à 20°C, supérieure à 80 Ncm$^3$/g de manière à adsorber au moins une partie du N$_2$O dudit flux et à produire un flux gazeux purifié, et
   (b) on récupère un flux gazeux purifié contenant une teneur en N$_2$O, inférieure à la teneur en N$_2$O du flux gazeux d'alimentation mis en contact avec l'adsorbant principal à l'étape a).

2. Procédé selon la revendication 1, **caractérisé en ce que** le flux gazeux d'alimentation mis en contact avec l'adsorbant principal à l'étape a) contient au moins 30% en volume de N$_2$O et de préférence au moins 40% en volume de N$_2$O.

3. Procédé selon l'une des revendications précédentes, **caractérisé en ce que** le flux gazeux d'alimentation mis en contact avec l'adsorbant principal à l'étape a) contient moins de 80% en volume de N$_2$O, de préférence moins de 70% en volume de N$_2$O.

4. Procédé selon l'une des revendications précédentes, **caractérisé en ce que** la zéolite est de type A, X, LSX, Mordénite, Offretite, Chabazite, Clinoptilolite ou Erionite.

**5.** Procédé selon l'une des revendications précédentes, **caractérisé en ce que** la zéolithe est échangée par un ou plusieurs cations métalliques choisis parmi $Na^+$, $Li^+$, $K^+$, $Ca^{2+}$, $Mg^{2+}$ et $Ba^{2+}$, et associés ou non à des métaux de transition, de préférence choisis parmi l'argent, le zinc ou le cuivre.

**6.** Procédé selon l'une des revendications précédentes, **caractérisé en ce que** l'adsorbant principal a une capacité d'adsorption à 1 bar et à 20°C du $N_2O$ supérieure à 90 $Ncm^3/g$, de préférence entre 90 et 120 $Ncm^3/g$.

**7.** Procédé selon l'une des revendications précédentes, **caractérisé en ce que** le flux gazeux d'alimentation contient également de la vapeur d'eau, du $CO_2$ et/ou de l'argon et/ou de l'azote.

**8.** Procédé selon l'une des revendications précédentes, **caractérisé en ce que** la capacité d'adsorption du $CO_2$ de l'adsorbant principal mesurée à 0.04 bar et à 20°C est supérieure ou égale à 30 $Ncm^3/g$, de préférence supérieure à 50 $Ncm^3/g$.

**9.** Procédé selon l'une des revendications précédentes, **caractérisé en ce qu'**il comporte, en outre, préalablement à l'étape a) une mise en contact du flux gazeux d'alimentation avec un adsorbant secondaire comprenant de l'alumine de manière à éliminer au moins une partie d'$H_2O$ et/ou du $CO_2$.

**10.** Procédé selon l'une des revendications précédentes, **caractérisé en ce que** le débit du flux gazeux d'alimentation à purifier est compris entre 1 et 30 Nlitres/min, de préférence entre 8 et 15 Nlitres/min.

**11.** Procédé selon la revendication 1, **caractérisé en ce que** l'adsorbant principal a une cinétique d'adsorption comprise entre 2 et 10 $sec^{-1}$.

**Patentansprüche**

**1.** Verfahren zur Reinigung eines Zufuhrgasflusses, der von einem Menschen oder einem Tier ausgeatmet wird und der mindestens 20 Vol.-% $N_2O$ und Sauerstoff enthält, wobei:

(a) der Zufuhrgasfluss bei einer Temperatur zwischen 20 °C und 40 °C und einem Druck zwischen 0,80 bar und 1,30 bar mit einem Hauptadsorptionsmittel in Kontakt gebracht wird, das mindestens einen Zeolithen umfasst, der zu mehr als 50 % mit einem oder mehreren Metallkationen ausgetauscht wurde, und das eine Adsorptionskinetik zwischen 2 und 20 $s^{-1}$, die bei 25 °C und 1 bar durch Messung der Durchbruchskurve ausgehend von einem $O_2/N_2O$-Gemisch, das mindestens 10 Vol.-% $N_2O$ enthält, mit einer Kinetikkonstante gemäß der linearen Antriebskraft bestimmt wird, und eine $N_2O$-Adsorptionskapazität von mehr als 80 $N.cm^3/g$, die bei 1 bar und 20 °C gemessen wird, aufweist, so dass mindestens ein Teil des $N_2O$ des Flusses adsorbiert und ein gereinigter Gasfluss produziert wird, und

(b) ein gereinigter Gasfluss zurückgewonnen wird, der einen $N_2O$-Gehalt enthält, der kleiner als der $N_2O$-Gehalt des Zufuhrgasflusses ist, der im Schritt a) mit dem Hauptadsorptionsmittel in Kontakt gebracht wurde.

**2.** Verfahren nach Anspruch 1, **dadurch gekennzeichnet, dass** der Zufuhrgasfluss, der im Schritt a) mit dem Hauptadsorptionsmittel in Kontakt gebracht wurde, mindestens 30 Vol.-% $N_2O$ und vorzugsweise mindestens 40 Vol.-% $N_2O$ enthält.

**3.** Verfahren nach einem der vorhergehenden Ansprüche, **dadurch gekennzeichnet, dass** der Zufuhrgasfluss, der im Schritt a) mit dem Hauptadsorptionsmittel in Kontakt gebracht wurde, mindestens 80 Vol.-% $N_2O$, vorzugsweise mindestens 70 Vol.-% $N_2O$ enthält.

**4.** Verfahren nach einem der vorhergehenden Ansprüche, **dadurch gekennzeichnet, dass** der Zeolith vom Typ A, X, LSX, Mordenit, Offretit, Chabazit, Clinoptilolit oder Erionit ist.

**5.** Verfahren nach einem der vorhergehenden Ansprüche, **dadurch gekennzeichnet, dass** der Zeolith mit einem oder mehreren Metallkationen ausgetauscht wird, die aus $Na^+$, $Li^+$, $K^+$, $Ca^{2+}$, $Mg^{2+}$ und $Ba^{2+}$ ausgewählt sind und gegebenenfalls mit Übergangsmetallen verbunden sind, die vorzugsweise aus Silber, Zink oder Kupfer ausgewählt sind.

**6.** Verfahren nach einem der vorhergehenden Ansprüche, **dadurch gekennzeichnet, dass** das Hauptadsorptionsmittel eine $N_2O$-Adsorptionskapazität von mehr als 90 $N.cm^3/g$, vorzugsweise zwischen 90 und 120 $N.cm^3/g$ bei 1 bar und 20 °C hat.

**7.** Verfahren nach einem der vorhergehenden Ansprüche, **dadurch gekennzeichnet, dass** der Zufuhrgasfluss außerdem Wasserdampf, $CO_2$ und/oder Argon und/oder Stickstoff enthält.

**8.** Verfahren nach einem der vorhergehenden Ansprüche, **dadurch gekennzeichnet, dass** die $CO_2$-Adsorptionskapazität des Hauptadsorptionsmittels, die bei 0,04 bar und 20 °C gemessen wird, größer gleich 30 $N.cm^3/g$, vorzugsweise größer als 50 $N.cm^3/g$ ist.

**9.** Verfahren nach einem der vorhergehenden Ansprüche, **dadurch gekennzeichnet, dass** es außerdem vor dem Schritt a) ein Inkontaktbringen des Zufuhrgasflusses mit einem sekundären Adsorptionsmittel umfasst, das Aluminiumoxid umfasst, um mindestens einen Teil des $H_2O$ und/oder des $CO_2$ zu entfernen.

10. Verfahren nach einem der vorhergehenden Ansprüche, **dadurch gekennzeichnet, dass** der Durchsatz des zu reinigenden Zufuhrgasflusses zwischen 1 und 30 N.Liter/min, vorzugsweise zwischen 8 und 15 N.Liter/min liegt.

11. Verfahren nach Anspruch 1, **dadurch gekennzeichnet, dass** das Hauptadsorptionsmittel eine Adsorptionskinetik zwischen 2 und 10 $s^{-1}$ hat.

**Claims**

1. Method for purifying a gaseous supply flow breathed out by a human or animal, containing at least 20 % by volume of $N_2O$ and oxygen, wherein:

    (a) the gaseous supply flow is brought into contact, at a temperature of between 20°C and 40°C and at a pressure of between 0.80 bar and 1.30 bar, with a principal adsorbent, comprising at least one zeolite more than 50% exchanged by one or more metal cations having adsorption kinetics of between 2 and 20 $sec^{-1}$ determined at 25°C and 1 bar by measuring the breakthrough curve from an $O_2/N_2O$ mixture containing less than 10% by volume of $N_2O$, with a kinetic constant according to the Linear Driving Force, and an adsorption capacity for $N_2O$ measured at 1 bar and at 20°C which is greater than 80 $Ncm^3/g$ so as to absorb at least some of the $N_2O$ of said flow and produce a purified gaseous flow, and
    (b) a purified gaseous flow is recovered, containing a content of $N_2O$ that is less than the content of $N_2O$ of the gaseous supply flow brought into contact with the principal adsorbent in step a).

2. Method according to claim 1, **characterised in that** the gaseous supply flow brought into contact with the principal adsorbent in step a) contains at least 30% by volume of $N_2O$ and preferably at least 40% by volume of $N_2O$.

3. Method according to one of the preceding claims, **characterised in that** the gaseous supply flow brought into contact with the principal adsorbent in step a) contains less than 80% by volume of $N_2O$, preferably less than 70% by volume of $N_2O$.

4. Method according to one of the preceding claims, **characterised in that** the zeolite is of the A, X, LSX, modenite, offretite, chabazite, clinoptilolite or erionite type.

5. Method according to one of the preceding claims, **characterised in that** the zeolite is exchanged by one or more metal cations selected from among $Na^+$, $Li^+$, $K^+$, $Ca^{2+}$, $Mg^{2+}$ and $Ba^{2+}$, which may or may not be combined with transition metals preferably selected from among silver, zinc or copper.

6. Method according to one of the preceding claims, **characterised in that** the principal adsorbent has an adsorption capacity at 1 bar and 20 °C of $N_2O$ which is greater than 90 $Ncm^3/g$, preferably between 90 and 120 $Ncm^3/g$.

7. Method according to one of the preceding claims, **characterised in that** the gaseous supply flow also contains water vapour, $CO_2$ and/or argon and/or nitrogen.

8. Method according to one of the preceding claims, **characterised in that** the adsorption capacity of the $CO_2$ of the principal adsorbent measured at 0.04 bar and at 20°C is greater than or equal to 30 $Ncm^3/g$, preferably greater than 50 $Ncm^3/g$.

9. Method according to one of the preceding claims, **characterised in that** it further comprises, prior to step a), contacting the gaseous supply flow with a secondary adsorbent comprising alumina so as to eliminate at least some of the $H_2O$ and/or $CO_2$.

10. Method according to one of the preceding claims, **characterised in that** the flow rate of the gaseous supply flow that is to be purified is between 1 and 30 Nlitres/min, preferably between 8 and 15 Nlitres/min.

11. Method according to claim 1, **characterised in that** the principal adsorbent has absorption kinetics of between 2 and 10 $sec^{-1}$.

**Figure 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4259303 A
- WO 9925461 A
- US 2006008401 A
- FR 2773144 A
- JP 2006142160 A
- EP 0698411 A
- JP 10165818 A
- EP 0995477 A
- US 4355637 A
- WO 0226355 A